# Europäisches Patentamt
# European Patent Office
# Office européen des brevets

(19)

(11) Veröffentlichungsnummer: **0 200 023**
**B1**

(12)

# EUROPÄISCHE PATENTSCHRIFT

(45) Veröffentlichungstag der Patentschrift:
**04.01.89**

(21) Anmeldenummer: **86104590.4**

(22) Anmeldetag: **04.04.86**

(51) Int. Cl.⁴: **B 01 J 31/40,** C 07 C 53/08,
C 07 C 53/12, C 07 C 69/16

(54) **Verfahren zur Reinigung und Rückgewinnung der bei der Carbonylierung von Methylacetat und/oder Dimethylether anfallenden verunreinigten Katalysatorlösung.**

(30) Priorität: **29.04.85 DE 3515396**

(43) Veröffentlichungstag der Anmeldung:
**05.11.86 Patentblatt 86/45**

(45) Bekanntmachung des Hinweises auf die Patenterteilung:
**04.01.89 Patentblatt 89/1**

(84) Benannte Vertragsstaaten:
**AT BE CH DE FR GB IT LI NL**

(56) Entgegenhaltungen:
**EP-A-0 073 922**
**EP-A-0 128 439**

(73) Patentinhaber: **HOECHST AKTIENGESELLSCHAFT,
Postfach 80 03 20, D-6230 Frankfurt am Main 80
(DE)**

(72) Erfinder: **Erpenbach, Heinz, Dr., Oberbuschweg 22,
D-5000 Köln (DE)**
Erfinder: **Gehrmann, Klaus Dr., Geschwister-
Scholl- Strasse 32, D-5042 Erftstadt (DE)**
Erfinder: **Lork, Winfried, Siegfried- von-
Westerburg- Strasse 14, D-5042 Erftstadt (DE)**
Erfinder: **Prinz, Peter, Von- Geyr- Ring 104, D-5030
Hürth (DE)**

LIBER, STOCKHOLM 1989

**Beschreibung**

Die Erfindung betrifft ein Verfahren zur Reinigung und Rückgewinnung der bei der Carbonylierung von Methylacetat und/ oder Dimethylether anfallenden verunreinigten Katalysatorlösung, welche Carbonylkomplexe des Rhodiums, quaternäre heterocyclische aromatische Stickstoffverbindungen oder quaternäre Organophosphorverbindungen als organische Promotoren, ggf. Verbindungen carbonylbildender Nichtedelmetalle als anorganische Promotoren, undestillierbare organische Verunreinigungen sowie Essigsäure, Essigsäureanhydrid und Ethylidendiacetat enthält.

Zur Durchführung von Hydroformylierungs- und Carbonylierungsverfahren wird als Edelmetallkatalysator Rhodium in Form vielfältiger komplexer Verbindungen eingesetzt. Da Rhodium aufgrund seiner geringen Verfügbarkeit ein sehr teures Edelmetall ist, wird über seine Rückgewinnung bzw. die Reinigung von Rhodiumkomplexen aus mit Rückständen verunreinigten Katalysatorsystemen bzw. Destillationssümpfen der oben angeführte Reaktionen mehrfach in der Literatur berichtet.

Den Unterschied im Löseverhalten des komplexen Rh-Katalysators und der anfallenden Teere belegen die Verfahren der EP-A-0 073 922 und 0 073 342. Beide Verfahren gehen bei der Reinigung und Rückgewinnung der bei der Carbonylierung von Methylacetat und/oder Dimethylether anfallenden verunreinigten Katalysatorlösung von einem Rhodium-Phosphonium- oder Rhodium-Ammonium-Carbonylkomplex als Katalysator aus. In beiden Fällen werden zunächst die flüchtigen Bestandteile aus der verunreinigten Katalysatorlösung entfernt. Gemäß EP-A-0 073 922 erfolgt die Entfernung der organischen Verunreinigungen aus dem verbleibenden Rückstand durch Extraktion mit aliphatischen Ethern. Gemäß EP-A-0 073 342 wird der Rückstand nach Abtrennen der flüchtigen Bestandteile zunächst mit Hilfe von Wasser in den wasserlöslichen organischen Promotor und in ein wasserunlösliches Gemisch aus Rhodiumcarbonylkomplex und organischen Verunreinigungen aufgetrennt. Aus dem wasserunlöslichen Rückstand werden sodann unter Verwendung aliphatischer Ether die organischen Verunreinigungen heraus gelöst. In beiden Fällen bleibt der Edelmetall-Carbonylkomplex erhalten und gelangt wieder in den Reaktionskreislauf, während die gebildeten Rückstände mit Ausbeuten von mehr als 90 % jeweils aus der Etherphase isoliert werden. Die Rhodium- bzw. Edelmetallausbeute erreicht Werte zwischen 98,8 und 99,6 %.

Die EP-A-0 128 439 beschreibt ein Verfahren, das es ermöglicht, den bei der Carbonylierung von Methylacetat und/oder Dimethylether eingesetzten und im Laufe des Prozesses verunreinigten Katalysatorkomplex so aufzuarbeiten, daß unter Extraktion der undestillierbaren organischen Verunreinigungen mit Ethylenglykoldialkylethern der Formel $R(OC_2H_4)_n$-OR (n = 1 - 4) das Edelmetall der Gruppe VIII in elementarer Form ausfällt und mit Ausbeuten > 99,9 %, bezogen auf eingesetztes Edelmetall, zurückgewonnen wird. Hier ist vor Wiedereinsatz des Rodiums in die Reaktion eine Überführung des elementaren Rhodiums in einen löslichen Rh-Carbonylkomplex erforderlich.

Der in jüngster Vergangenheit zu verzeichnende weitere steile Preisanstieg des Rhodiums gestattet nur seine verlustfreie Verwendung als Katalysator für großtechnische Verfahren. Dies gilt auch für die Reinigung und Rückgewinnung eines eingesetzten verunreinigten Rhodiumkatalysators und macht daher einen quantitativen Kreislauf unerläßlich. Dies erfordert eine ohne Zwischenstufen verlaufende, möglichst einfache Abtrennung des im Verfahren gebildeten organischen Rückstandes aus der Katalysatorlösung ohne jeden Rh-Verlust und den direkten Wiedereinsatz des gereinigten Katalysatorsystems in die Reaktion.

Die Verfahren der EP-A-0 073 922, 0 073 342 und 0 128 439 erfüllen diese Forderungen bereits weitgehend, jedoch nicht quantitativ. Während in den ersten beiden Fällen bei Rh-Ausbeuten von höchstens 99,8 % der Rh-Carbonylkomplex unzerstört bleibt, gelingt es in der zuletzt zitierten Anmeldung, die Rh-Ausbeute nach der Reinigung noch auf Werte > 99,9 % zu steigern. Doch hierbei fällt das Rhodium, wie erwähnt, in metallischer Form an und muß durch zusätzliche Maßnahmen in die Komplexform überführt werden.

Die Summe der Forderungen wird nun durch die vorliegende Erfindung erfüllt, die ein Verfahren beschreibt, das es überraschenderweise ermöglicht, die bei der Carbonylierung von Methylacetat und/oder Dimethylether eingesetzte und im Laufe des Prozesses verunreinigte Katalysatorlösung durch einfache Destillation und Extraktion so aufzuarbeiten, daß eine Abtrennung der undestillierbaren organischen Verunreinigungen aus der Katalysatorlösung ohne Rhodiumverlust und ohne Zerstörung des Rh-Carbonylkomplexes sowie des Promotors erfolgen kann. Rh-Carbonylkomplex und Promotorsalz können so ohne aufwendige Rekombinationsmaßnahmen dem Carbonylierungsprozeß wieder zugeführt werden. Gleichzeitig wird durch den Kreislaufbetrieb der zur Aufarbeitung eingesetzten Extraktionsmittel eine zusätzliche Umweltbelastung durch Abfallstoffe vermieden. Nur die im Prozeß gebildeten undestillierbaren organischen Verunreinigungen werden ausgeschleust und können entsprechend dem Stand der Technik beseitigt werden.

Im einzelnen ist das Verfahren der Erfindung nunmehr dadurch gekennzeichnet, daß man der verunreinigten Katalysatorlösung in einer ersten Verfahrensstufe durch Extraktion mit Dialkylethern mit 1 - 4 C-Atomen die organischen Verunreinigungen sowie Essigsäure, Essigsäureanhydrid und Ethylidendiacetat entzieht und die Etherphase von einem ausgefallenen, promotorhaltigen Katalysatorkomplex abtrennt; daß man die Etherphase in einer zweiten Verfahrensstufe mit Jod und/oder Methyljodid behandelt, weiteren ausgefallenen, promotorhaltigen Katalysatorkomplex abtrennt und mit dem gereinigten Katalysatorkomplex der ersten Stufe vereinigt; daß man die Etherphase durch Destillation auftrennt, den zurückgewonnenen Dialkylether erneut zur Extraktion einsetzt, aus dem zurückgewonnenen Gemisch aus Essigsäure, Essigsäureanhydrid und Ethylidendiacetat und dem vereinigten gereinigten Katalysatorkomplex erneut frische Katalysatorlösung bereitet und die als Rückstand der Etherphasendestillation verbleibenden organischen Verunreinigungen ausschleust; und daß man die gereinigte Katalysatorlösung destillativ von restlichem Dialkylether befreit.

Das Verfahren der Erfindung kann weiterhin bevorzugt und wahlweise dadurch gekennzeichnet sein, daß man

a) die Extraktion der Katalysatorlösung mit Dialkylether und die Behandlung der Etherphase mit Jod und/oder Methyljodid bei Temperaturen von 20 - 140°C und Drücken von 1 bis 30 bar durchführt;

b) je Gewichtsteil verunreinigter Katalysatorlösung 0,5 - 20 Gewichtsteile Dialkylether einsetzt;

c) je Gewichtsteil Dialkylether 0,00005 - 0,01 Gewichtsteile Jod und/oder Methyljodid einsetzt;

d) die erste und zweite Verfahrensstufe verbindet, indem man die verunreinigte Katalysatorlösung mit Jod und/oder Methyljodid und Dialkylether behandelt und die Etherphase vom ausgefallenen promotorhaltigen Katalysatorkomplex abtrennt;

e) je Gewichtsteil verunreinigter Katalysatorlösung 0,0001 - 0,01 Gewichtsteile Jod und/oder Methyljodid einsetzt.

Zur Herkunft der verunreinigten Katalysatorlösung ist zu sagen, daß die aus einen Carbonylierungsreaktor abfließende Reaktionsmischung destillativ in die gewünschten Endprodukte, besonders Essigsäureanhydrid, Essigsäure und/oder Ethylidendiacetat, und nichtumgesetzte, im Kreislauf geführte Ausgangsstoffe einerseits sowie die als Sumpfprodukt anfallende und in Kreislauf geführte Katalysatorlösung andererseits aufgetrennt wird. Ein Teilstrom dieser mit der Zeit durch undestillierbare organische Produkte verunreinigten Katalysatorlösung, welche je nach Verfahrensbedingungen bis zu 50 Masse-% an Essigsäureanhydrid, Essigsäure und/oder Ethylidendiacetat enthalten kann, wird aus den Kreislauf der Katalysatorlösung entnommen und der Reinigung zugeführt. Die verunreinigte Katalysatorlösung enthält das Edelmetall Rhodium als Carbonylkomplex wie z. B. $[CH_3P(C_4H_9)_3]Rh(CO)I_4$ oder $[CH_3P(C_4H_9)_3]Rh(CO)_2I_2$.

Die Katalysatorlösung enthält ferner als organische Promotoren bevorzugt einen oder mehrere der folgenden heterocyclischen aromatischen Stickstoffverbindungen oder Organophosphorverbindungen:

1. N-Methylpyridiniumjodid, N,N-Dimethylimidazoliumjodid, N-Methyl-3-picoliniumjodid, N-Methyl-2,4-lutidiniumjodid, N-Methyl-3,4-lutidiniumjodid, N-Methyl-chinoliniumjodid;

2. Tri-n-butyl-methyl-phosphoniumjodid, Trioctyl-methylphosphoniumjodid, Trilauryl-methyl-phosphoniumjodid, Triphenyl-methyl-phosphoniumjodid.

Schließlich kann die Katalysatorlösung als anorganische Promotoren Verbindungen der carbonylbildenden Nichtedelmetalle Ce, Ti, Zr, Hf, Ge, Sn, Pb, V, Nb, Ta, As, Sb, Bi, Cr, Mo, W, Mn, Re, Fe, Co, Ni enthalten.

Die ausgeschleuste verunreinigte Katalysatorlösung wird mit dem Dialkylether bevorzugt bei 20 - 140°C (1 - 30 bar) extrahiert. Hierbei werden die bei der Reaktion gebildeten undestillierbaren organischen Verunreinigungen sowie die in der Katalysatorlösung befindlichen Anteile Essigsäureanhydrid, Essigsäure und/oder Ethylidendiacetat gelöst, während der Rh-Carbonylkomplex mit dem oder den Promotoren als ungelöste Katalysatorphase zurückbleibt. Die abgezogene Etherphase wird sodann unter Zusatz von Jod und/oder Methyljodid bei bevorzugt 20 - 140°C (1-30 bar) nachbehandelt, wobei ein rhodiumhaltiger Niederschlag ausfällt. Dieser Niederschlag wird abgetrennt und der gereinigten Katalysatorphase zugesetzt. Nach Abtrennen des Niederschlags aus der Etherphase wird der Ether durch Destillation zurückgewonnen und in die Extraktion zurückgeführt. Anschließend werden Essigsäureanhydrid, Essigsäure und/oder Ethylidendiacetat redestilliert, wobei die undestillierbaren organischen Verunreinigungen als Rückstand anfallen. Die redestillierten Reaktionsprodukte werden zur Lösung der gereinigten Katalysatorphase (Rh-Carbonylkomplex und Promotorsalz) verwendet und nach Entfernen von u. U. noch anhaftendem Ether in den Carbonylierungsprozeß zurückgeführt. Die undestillierbaren organischen Verunreinigungen werden z. B. in einer Verbrennungsanlage vernichtet.

Das Verfahren der Erfindung kann in sowohl kontinuierlicher als auch diskontinuierlicher Betriebsweise durchgeführt werden.

**Beispiel 1**

Zur Entfernung der organischen Verunreinigungen werden dem Katalysatorkreislauf der Dimethylethercarbonylierung 1000 g Katalysatorlösung der Zusammensetzung 6,42 M-% Rhodiumcarbonylkomplex [CH$_3$P(C$_4$H$_9$)$_3$] [Rh(CO)$_2$I$_2$] (entsprechend 10,5 g = 1,05 M-% Rh), 67,38 M-% Methyl-tri-n-butylphosphoniumjodid, 6,2 M-% organische Verunreinigungen, 6,6 M-% Essigsäure, 13,3 M-% Essigsäureanhydrid und 0,1 M-% Ethylidendiacetat entnommen und unter Rühren innerhalb von 30 min in 3000 g Diisopropylether (S.P. = 67,5°C) eingetropft. Nach weiteren 30 min Rühren wird die Etherphase vom ausgefallenen promotorhaltigen Katalysatorkomplex abgetrennt, mit 0,8 g elementarem Jod versetzt und 2 h bei 70°C unter Rückfluß gekocht. Dabei fällt ein Rh-haltiger Niederschlag aus. Dieser Niederschlag wird abfiltriert und zum ausgefallenen Katalysatorkomplex gegeben. Die filtrierte Etherphase wird sodann in 2980 g Diisopropylether, 65 g Essigsäure, 131 g Essigsäureanhydrid, 1 g Ethylidendiacetat und 55,9 g undestillierbare organische Verunreinigungen als teerartiger Rückstand (Rh-Gehalt 0,005 M-%) destillativ aufgetrennt. Mit den gewonnenen Gemisch aus Essigsäure, Ethylidendiacetat und Essigsäureanhydrid wird der gereinigte promotorhaltige Katalysatorkomplex unter Erwärmen und ggf. Einleiten von CO aufgelöst. Dabei werden noch 20 g Diisopropylether als Destillat entfernt, während 945 g gereinigte Katalysatorlösung mit einem Rh-Gehalt von 10,5 g verbleiben und dem Katalysatorkreislauf wieder zugesetzt werden. Die beiden Etherdestillate werden vereinigt und zur erneuten Extraktion verwendet. Die Rückführungsrate des Rhodiums in den Carbonylierungsprozeß beträgt nach der Reinigung der abgezogenen verunreinigten Katalysatorlösung 99,97 %.

**Beispiel 2 (Vergleichsbeispiel)**

Zur Entfernung der organischen Verunreinigungen werden 1000 g verunreinigte Katalysatorlösung der Zusammensetzung gemäß Beispiel 1 dem Katalysatorkreislauf der Dimethylethercarbonylierung entnommen und unter Rühren innerhalb von 30 min in 3000 g Diisopropylether eingetropft. Nach weiteren 30 min Rühren wird die Etherphase vom ausgefallenen, promotorhaltigen Katalysatorkomplex durch Filtration abgezogen. Die filtrierte Etherphase wird ohne weitere Jodbehandlung in 2980 g Diisopropylether, 197 g Essigsäure-Essigsäureanhydrid-Ethylidendiacetat-Gemisch und 56,1 g undestillierbare organische Verunreinigungen als Rückstand (Rh-Gehalt 0,45 M-%) destillativ aufgetrennt. Der gereinigte ausgefallene Katalysatorkomplex wird mit dem aus der Etherphase gewonnenen AcOH-Ac$_2$0-EDA-Gemisch unter Erwärmen aufgelöst, wobei gleichzeitig noch 20 g Diisopropylether abdestillieren. Es werden 944 g gereinigte Katalysatorlösung mit einem Rh-Gehalt von 10,25 g zum Niedereinsatz erhalten. Die Rückführungsrate des Rhodiums in den Carbonylierungsprozeß beträgt nur 97,6 %.

**Beispiel 3**

Zur Entfernung der organischen Verunreinigungen werden dem Katalysatorkreislauf der Methylacetatcarbonylierung 1000 g Katalysatorlösung der Zusammensetzung 5,6 M-% Rh-Carbonylkomplex C$_5$H$_9$N$_2$ [Rh(CO)$_2$I$_2$] (entsprechend 11,3 g = 1,13 M-% Rh), 70,0 M-% N,N-Dimethylimidazoliumjodid (C$_5$H$_9$N$_2$I), 8,1 M-% organische Verunreinigungen, 16,3 M-% Essigsäure, Essigsäureanhydrid und Ethylidendiacetat entnommen und unter Rühren innerhalb von 15 min in 4000 g Diethylether (S.P. = 34,6°C) eindosiert. Nach weiteren 30 min Rühren wird die Etherphase vom ausgefallenen, promotorhaltigen Katalysatorkomplex abgezogen, mit 4 g Methyljodid versetzt und 2 h bei 36°C unter Rückfluß gekocht. Es fällt ein Rh-haltiger Niederschlag aus, der nach Abtrennung dem ausgefallenen Katalysatorkomplex zugeschlagen wird. Die vom Rh-haltigen Niederschlag befreite Etherphase wird danach in 3930 g Diethylether, 160 g Essigsäure-Essigsäureanhydrid-EDA-Gemisch und 75,1 g auszuschleusende organische Verunreinigungen (Rh-Gehalt 0,007 M-%) zerlegt. Während die organischen Verunreinigungen zur Verbrennung gegeben werden, dient das erhaltene Essigsäure-Essigsäureanhydrid-EDA-Gemisch als Lösemittel für den vereinigten gereinigten Katalysatorkomplex. Nach Auflösen des promotorhaltigen Katalysatorkomplexes und Abdestillieren von 70 g Diethylether werden 929 g gereinigte Katalysatorlösung mit einem Rh-Gehalt von 11,3 g dem Carbonylierungsprozeß wieder zugeführt. Die Rückführungsrate des Rhodiums beträgt 99,95 %. Die beiden Etherdestillate werden vereinigt und zur erneuten Extraktion verwendet.

**Beispiel 4**

Zur Entfernung der organischen Verunreinigungen werden aus dem Katalysatorkreislauf der Methylacetatcarbonylierung 1000 g Katalysatorlösung der Zusammensetzung 6,0 M-% Rh-Carbonylkomplex [CH$_3$P(C$_4$H$_9$)$_3$] [Rh(CO)$_2$I$_2$] (entsprechend 9,8 g = 0,98 M-% Rh),

4

62,1 M-% Methyl-tri-n-butylphosphonium jodid, 7,3 M-% organische Verunreinigungen, 24,6 M-% Essigsäure und Essigsäureanhydrid abgezogen, mit 6 g elementarem Jod versetzt, 0,5 h unter Rühren auf 110°C erwärmt und dann unter Rühren in 3500 g Di-n-propylether (S.P. = 91°C) eingegeben. Nach weiteren 30 min Rühren bei 90°C werden der ausgefallene, promotorhaltige Katalysatorkomplex und die Etherphase getrennt. Aus der Etherphase werden 3450 g Di-n-propylether, 242 g Essigsäure und Essigsäureanhydrid sowie 67,2 g undestillierbare organische Verunreinigungen (Rh-Gehalt 0,007 M-%) gewonnen. Während die organischen Verunreinigungen verworfen werden, dient das aus dem Etherextrakt gewonnene Essigsäure-Essigsäureanhydrid-Gemisch zum Auflösen des gereinigten promotorhaltigen Katalysatorkomplexes. Nach der destillativen Entfernung von 50 g restlichem Di-n-propylether werden 939 g gereinigte Katalysatorlösung mit einem Rh-Gehalt von 9,8 g dem Carbonylierungsprozeß zugesetzt, wobei sich eine Rh-Rückführungsrate von 99,95 % ergibt. Die vereinigten Etherdestillate werden in die Extraktionsstufe zurückgeführt

**Beispiel 5**

Zur Entfernung der organischen Verunreinigungen werden dem Katalysatorkreislauf der Dimethylethercarbonylierung 1000 g Katalysatorlösung mit der Zusammensetzung 8,08 M-% Rh-Carbonylkomplexe [CH$_3$P(C$_4$H$_9$)$_3$] [Rh(CO)$_2$I$_2$] und [CH$_3$P(C$_4$H$_9$)$_3$] [RhCOI$_4$] im Verhältnis 1 : 1 (entsprechend 11,2 g = 1,12 M-% Rh), 49,22 M-% Methyl-tri-n-butylphosphoniumjodid, 5,9 M-% organische Verunreinigungen, 36,8 M-% Essigsäure, Essigsäureanhydrid und Ethylidendiacetat entnommen und unter Rühren in ein Gemisch aus 2,5 g Jod und 2500 g Ethyl-n-butyl-ether (S.P. = 91,4°C) innerhalb von 30 min eindosiert. Die Temperatur im Extraktionsgefäß wird dabei auf 90°C gehalten. Nach weiteren 30 min Rühren erfolgt die Abtrennung der Etherphase vom promotorhaltigen Katalysatorkomplex und ihre destillative Zerlegung in 2450 g Ethyl-n-butylether, 360 g Essigsäure-, Essigsäureanhydrid- und EDA-Gemisch sowie 52,5 g organische Verunreinigungen (Rh-Gehalt 0,008 M-%), welche verworfen werden. Nach Vereinigung des gereinigten promotorhaltigen Katalysatorkomplexes mit dem aus der Etherphase zurückgewonnenen AcOH-Ac$_2$O-EDA-Gemisch werden noch 50 g restlicher Ethyl-n-butylether destillativ abgetrennt, so daß anschließend 950 g gereinigte Katalysatorlösung mit einem Rh-Gehalt von 11,2 g dem Katalysatorkreislauf zudosiert werden können. Die Rückführungsrate des Rhodiums ergibt 99,96

%. Die vereinigten Etherdestillate werden der Extraktionsstufe erneut zugeführt.

**Patentansprüche**

1. Verfahren zur Reinigung und Rückgewinnung der bei der Carbonylierung von Methylacetat und/oder Dimethylether anfallenden verunreinigten Katalysatorlösung, welche Carbonylkomplexe des Rhodiums, quaternäre heterocyclische aromatische Stickstoffverbindungen oder quaternäre Organophosphorverbindungen als organische Promotoren, gegebenenfalls Verbindungen carbonylbildender Nichtedelmetalle als anorganische Promotoren, undestillierbare organische Verunreinigungen sowie Essigsäure, Essigsäureanhydrid und Ethylidendiacetat enthält, dadurch gekennzeichnet, daß man der verunreinigten Katalysatorlösung in einer ersten Verfahrensstufe durch Extraktion mit Dialkylethern mit 1 - 4 C-Atomen die organischen Verunreinigungen sowie Essigsäure, Essigsäureanhydrid und Ethylidendiacetat entzieht und die Etherphase von einem ausgefallenen, promotorhaltigen Katalysatorkomplex abtrennt; daß man die Etherphase in einer zweiten Verfahrensstufe mit Jod und/oder Methyljodid behandelt, weiteren ausgefallenen, promotorhaltigen Katalysatorkomplex abtrennt und mit dem gereinigten Katalysatorkomplex der ersten Stufe vereinigt; daß man die Etherphase durch Destillation auftrennt, den zurückgewonnenen Dialkylether erneut zur Extraktion einsetzt, aus dem zurückgewonnenen Gemisch aus Essigsäure, Essigsäureanhydrid und Ethylidendiacetat und dem vereinigten gereinigten Katalysatorkomplex erneut frische Katalysatorlösung bereitet und die als Rückstand der Etherphasendestillation verbleibenden organischen Verunreinigungen ausschleust; und daß man die gereinigte Katalysatorlösung destillativ von restlichem Dialkylether befreit.

2. Verfahren nach Anspruch 1, dadurch gekennzeichnet, daß man die Extraktion der Katalysatorlösung mit Dialkylether und die Behandlung der Etherphase mit Jod und/oder Methyljodid bei Temperaturen von 20 - 140°C und Drücken von 1 bis 30 bar durchführt.

3. Verfahren nach Anspruch 1 oder 2, dadurch gekennzeichnet, daß man je Gewichtsteil verunreinigter Katalysatorlösung 0,5 - 20 Gewichtsteile Dialkylether einsetzt.

4. Verfahren nach einem der Ansprüche 1 - 3, dadurch gekennzeichnet, daß man je Gewichtsteil Dialkylether 0,00005 - 0,01 Gewichtsteile Jod und/oder Methyljodid einsetzt.

5. Verfahren nach einen der Ansprüche 1 - 4, dadurch gekennzeichnet, daß man die erste und zweite Verfahrensstufe verbindet, indem man die verunreinigte Katalysatorlösung mit Jod und/oder Methyljodid und Dialkylether behandelt und die Etherphase vom ausgefallenen, promotorhaltigen Hatalysatorkomplex abtrennt.

6. Verfahren nach Anspruch 5, <u>dadurch gekennzeichnet,</u> daß man je Gewichtsteil verunreinigter Katalysatorlösung 0,0001 - 0,01 Gewichtsteile Jod und/oder Methyljodid einsetzt.

## Claims

1. A process for the purification and recovery of the contaminated catalyst solution formed in the carbonylation of methyl acetate and/or dimethyl ether containing carbonyl complexes of rhodium, quaternary heterocyclic aromatic nitrogen compounds or quaternary organophosphorus compounds as organic promoters, possibly compounds of carbonyl-forming base metals as inorganic promoters, undistillable organic impurities and also acetic acid, acetic anhydride and ethyl idene diacetate, which comprises stripping the contaminated catalyst solution in a first process stage by extraction with dialkyl ethers having 1 - 4 carbon atoms of the organic impurities and also of acetic acid, acetic anhydride and ethylidene diacetate and separating off the ether phase from a precipitated, promoter-containing catalyst complex; treating the ether phase in a second process stage with iodine and/or methyl iodide, separating off further precipitated, promoter-containing catalyst complex and combining it with the purified catalyst complex of the first stage; separating the ether phase by distillation, using the recovered dialkyl ether again for extraction, again preparing fresh catalyst solution from the recovered mixture consisting of acetic acid, acetic anhydride and ethylidene diacetate and the combined purified catalyst complex and discharging the organic impurities remaining as residue in the distillation of the ether phase; and freeing the purified catalyst solution by distillation from the remaining dialkyl ether.

2. The process as claimed in claim 1, wherein the extraction of the catalyst solution with dialkyl ether and the treatment of the ether phase with iodine and/or methyl iodide is carried out at temperatures of 20 - 140°C and pressures of 1 to 30 bar.

3. The process as claimed in claim 1 or 2, wherein 0.5 - 20 parts by weight of dialkyl ether are used per part by weight of contaminated catalyst solution.

4. The process as claimed in one of claims 1 to 3, wherein 0.00005 - 0.01 parts by weight of iodine and/or methyl iodide are used per part by weight of dialkyl ether.

5. The process as claimed in one of claims 1 to 4, where in the first and second process stages are combined by treating the contaminated catalyst solution with iodine and/or methyl iodide and dialkyl ether and separating off the ether phase from the precipitated, promoter-containing catalyst complex.

6. The process as claimed in claim 5, wherein 0.0001-0.01 parts by weight of iodine and/or methyl iodide are used per part by weight of contaminated catalyst solution.

## Revendications

1. Procédé pour purifier et récupérer la solution de catalyseur impure provenant de la carbonylation de l'acétate de méthyle et/ou de l'éther diméthylique, qui contient des complexes carbonylés du rhodium, des composés hétérocycliques aromatiques quaternaires azotés ou des composés organophosphorés quaternaires en tant qu'activateurs organiques, le cas échéant des composés de métaux non nobles formant des dérivés carbonylés en tant qu'activateurs minéraux, des impuretés organiques non distillables et de l'acide acétique, de l'anhydride acétique et du diacétate d'éthylidène, caractérisé en ce que, dans un premier stade opératoire, on élimine de la solution de catalyseur impure, par extraction à l'aide d'éthers dialkyliques en $C_1$ - $C_4$, les impuretés organiques et l'acide acétique, l'anhydride acétique et le diacétate d'éthylidène, et on sépare la phase éther d'un complexe catalyseur contenant l'activateur qui a précipité; dans un deuxième stade opératoire, on traite la phase éther par l'iode et/ou l'iodure de méthyle, on sépare les compléments du complexe de catalyseur contenant l'activateur qui a précipité et on les combine avec le complexe catalyseur purifié au premier stade opératoire; on sépare la phase éther par distillation, on réutilise l'éther dialkylique récupéré pour l'extraction, on prépare à partir du mélange récupéré d'acide acétique, d'anhydride acétique et de diacétate d'éthylidene et du complexe catalyseur purifié combiné une solution fraîche de catalyseur et on évacue les impuretés organiques restant en résidu de la distillation des phases éthers; et on débarrasse la solution de catalyseur purifiée de l'éther dialkylique résiduel par distillation.

2. Procédé selon la revendication 1, caractérisé en ce que l'extraction de la solution de catalyseur par l'éther dialkylique et le traitement de la phase éther par l'iode et/ou l'iodure de méthyle sont effectués à des températures de 20 à 140°C et des pressions de 1 à 30 bars.

3. Procédé selon la revendication 1 ou 2, caractérisé en ce que l'on utilise de 0,5 à 20 parties en poids d'éther dialkylique par partie en poids de la solution de catalyseur impure.

4. Procédé selon l'une des revendications 1 à 3, caractérisé en ce que l'on utilise de 0,00005 à 0,01 partie en poids d'iode et/ou d'iodure de méthyle par partie en poids d'éther dialkylique.

5. Procédé selon l'une des revendications 1 à 4, caractérisé en ce que l'on combine le premier et le deuxième stade opératoire en traitant la solution de catalyseur impure par l'iode et/ou l'iodure de méthyle et l'éther dialkylique et en séparant la phase éther du complexe catalyseur contenant l'activateur qui a précipité.

6. Procédé selon la revendication 5, caractérisé en ce que l'on utilise de 0,0001 à 0,1 partie en poids d'iode et/ou d'iodure de méthyle par partie en poids de la solution de catalyseur impure.